# EUROPEAN PATENT APPLICATION

(11) **EP 1 197 217 A1**
(43) Date of publication of application: **17.04.2002**
(21) Application number: 01123182.6
(22) Date of filing: 27.09.2001
(51) Int. Cl.: A61K 33/26, A61P 15/04

(54) **Use of sodium nitroprussiate in obstetrics and gynaecology**

(30) Priority: 29.09.2000 IT MI002117
(71) Applicant: Ibsa Institut Biochimique S.A., 6903 Lugano (CH)
(72) Inventor: Facchinetti, Fabio, 41013 Castelfranco Emilia (Modena) (IT)
(74) Representative: Gervasi, Gemma, Dr.

(57) **Abstract**

The present invention refers to the use of sodium nitroprussiate as a drug in the obstetric and gynaecological field. The use of said drug, preferably by the intracervical or vaginal way, causes a considerable increase in the distensibility, softness and penetrability of the uterine cervix, without producing the side effects usually associated with nitroderivatives of pharmaceutical use. The treatment described herein increases the efficacy and safety margin of several kinds of obstetric and gynaecological treatments, such as for example induced delivery or assisted procreation.

## Description

### Field of the invention

The present invention refers to the use of drugs in the obstetric and gynaecological field.

### Prior art

The discovery that various tissues produce nitric oxide (NO) was a source for considerable research on the possible involvement of said agent in physiological and pathophysiological processes, female reproduction and perinatalogy included (Neri et al., *Obstet.Gynecol.Surv*. 50,1995, 851; Rosselli et al., *Hum*.*Reprod*.*Update*, 4,1998, 3-24). This last involvement is of major importance for the development of drugs useful in obstetrics and gynaecology.

Evidence was provided that NO not only inhibits myometrium contractility (*Izumi et al*., *Am.J.Obstet.Gynecol*., 169, 1327), but can be involved in the control of the cervix ripening (Garfield et al. in *"Nitric Oxide, Cytochromes P450 and Sexual Steroid Hormones*" Springer, Berlin, 1997, pp.141-180). The activity of inducible NO synthase was detected in cervices of rats and guinea pigs, in which--unlike in the myometrium--it increases during labour (*Hum.Reprod*., II, 1996, 1755). Furthermore, in guinea pigs the local application of NO donors induces cervix ripening associated with ultrastructural and functional changes (Chwalisz et al., *Hum*.*Reprod*.,12, 1987, 2093-2101). NO may also produce effects through the mediation of prostaglandins, as evidenced by *in* vitro determinations (Ledingham et al., *Mol*.*Hum*.*Reprod*., 5, 1999, 973). A similar effect was observed in the woman. It was also reported that glyceryl trinitrate and isosorbide mononitrate (Thomson et al., *Lancet*, 352, 1998, 1093) administered by the vaginal way can reduce the cumulative force necessary to dilate the woman's cervix in the first trimester of pregnancy.

Tests performed so far on uterus dilatation revealed considerable limitations as to the extent and duration of the dilatative effect and caused by the onset of serious untoward side effects. In particular, all NO donors exhibit a considerable hypotensive activity, which is highly dangerous, e.g. during delivery, when mother and child need large blood and oxygen supply. Furthermore, the use of NO donors is connected with the onset of severe migraines, as reported in various clinical studies (e.g. Thomson et al., *Eur.J.Neurol*., 1, 1994, 73-80).

In view of the aforesaid limitations, the need for efficacious and safe drugs capable of inducing uterus softness is deeply felt.

### Summary

The present invention refers to the use of sodium nitroprussiate as a drug in the obstetric and gynaecological field. The use of said drug, preferably by the intracervical or vaginal way, causes a considerable increase in the uterine cervix distensibility, softness and penetrability, without producing the side effects commonly associated with the nitroderivatives of pharmaceutical use. The treatment described herein increases the efficacy and safety margin of several kinds of obstetric and gynaecological treatment, such as for example induced delivery or assisted procreation.

### Detailed description of the invention

It has surprisingly been found that sodium nitroprussiate can cause a remarkable increase in the rate of uterine cervix ripening, and in particular in the uterus distensibility, softness and penetrability. Furthermore, contrary to all expectations, the administration of this product produces neither the normal untoward side effects, such as hypotension, nor other side effects, such as migraine or pelvic cramps, which are brought about by the administration of nitroderivatives. Therefore, nitroprussiate has proven to be a drug exhibiting high efficacy and tolerability in the obstetric and gynaecological field.

The present invention refers to the use of sodium nitroprussiate in the preparation of drugs for obstetric and gynaecological application, particularly suitable for increasing the uterus distensibility, softness and penetrability. As illustrated by the examples reported herein, the treatment with nitroprussiate is useful in a great number of procedures requiring an increased uterine softness. Non-limiting examples of said procedures are artificial insemination, delivery, insertion of uterine devices, e.g. for contraceptive purposes, performance of gynaecological treatment or tests, such as for example hysteroscopy or revision of the uterine cavity, suction of the uterus, endometrial curettage and abortion induction.

Delivery induction at any time of gestation, including gestation at term, may be facilitated by eliminating the cervical resistance factor, without inducing inappropriate contractions, as produced by prostaglandins.

The termination of pregnancy in the first or second trimester may be carried out more effectively since the cervix, which is often insensitive to prostaglandins, represents the main obstacle. In the case of spontaneous abortion, either incomplete or missed, chemical ripening may allow the emptying of the uterus using a lower amount of anaesthetics, if any.

In all aforementioned cases, the increased uterine softness facilitates the access to the uterus itself as well as the intracervical motion. This favours all aforesaid kinds of treatment and increases their efficacy, safety, and speed, reduces the risk of lesions, inflammations or infections, increases the patient's comfort, and facilitates the doctor's work.

Sodium nitroprussiate is preferably administered by the intracervical or vaginal way, e.g. by applying ointments or gels once or twice depending on the intensity and persistence of the effect required. The aforesaid effects are obtained by application of a quantity of active ingredient (sodium nitroprussiate) of 1 mg to 10 mg, preferably approx. 5 mg.

The invention includes pharmaceutical compositions based on sodium nitrate, specifically prepared for intracervical or vaginal administration: examples of said compositions are ointments, or gels. Administration may be carried out once or several times, depending on intensity and duration of the desired effect. Effects as above are obtained administrating topically an amount of active principle (sodium nitroprussiate) comprised between 1 mg and 10 mg, preferably about 5 mg.

The invention comprises pharmaceutical compositions, specifically adapted for intracervical o vaginal administration, which are based on sodium nitroprussiate: examples for such compositions are ointments, gels, lavages, tablets, suppositories, medicated aids, etc. The concentration of nitroprussiate in said formulations varies depending on the cases: by way of example, concentrations may be of 0.5% to 3% by wt., preferably approx. 1%.

The following experimental examples are conveyed by way of indication, not of limitation, of the present invention.

### Experimental part

### Example 1: Assessment of the cervix softening by application of sodium nitroprussiate

### Procedure

Trials were conducted using 36 women who had been admitted to the hospital for voluntary interruption of pregnancy in the first trimester. In accordance with Italian law No. 194/78, the admittance criteria were the following: first pregnancy, pregnancy length of 9 to 12.5 weeks (determined on the basis of the last menstrual period and of the clinical test). Said women were subjected to emptying of the uterus under local anaesthesia. Exclusion criteria were: inability to understand the consent form, multiple pregnancy, previous uterine surgery, acknowledged allergy to drugs and any chronic disease requiring treatment. Eighteen women--after visualization of the cervix by means of a speculum--were randomised to receive a placebo gel or a gel based on 1 % nitroprussiate (equivalent to 5 mg) in the cervical canal, through a plastic catheter. While the patients were lying on their backs, the blood pressure was measured with an automatic apparatus (Dinamap® , Critikon, Tampa, FL, USA), immediately before, and 60, 120, 240 and 360 minutes after, treatment. Two and four hours after treatment and, again, two hours after the operation, the onset of adverse effects, such as cephalea, vomiting, palpitation and abdominal pains, was checked. The uterus was emptied approx. 6 hours after gel administration.

Once the treatment of the first group of women was completed, further 18 women were registered according to the same admittance and exclusion criteria. The second group of women was randomised to receive placebo gel or a gel based on 2% nitroprussiate (equivalent to 10 mg). Uterus suction was performed 3 hours after gel administration on the basis of the effects previously observed. The blood pressure was measured immediately before, and 60, 120 and 180 minutes after, treatment.

Once the paracervical block was obtained by injecting 2% Camocaina® (6 ml) (Astra Farmaceutici, Milan, Italy), the force necessary to dilate the cervix to 10 mm was recorded by means of a specific dynamometer, supplied by the Department of Clinical Physics and Bioengineering of the University of Glasgow (GB), connected with Hegar's dilators (Richardson et al., *J.Med.Eng.Technol*. 13, 1989, 220). Cervical measurements and pregnancy terminations were performed by an operator who ignored the treatment done.

Cervical resistance was the main result. The "resistance of the cervix at 8 mm" was calculated also by adding the peak force necessary to dilate the cervix from 4 to 8 mm. In a previous study, this parameter was reduced by 30% with the use of NO donors instead of the placebo.

Comparisons of the cervical data between the various subjects were performed by analyses of variance (ANOVA). Blood pressure variations after treatment were computed with the baseline value in a subject using multiple ANOVA (MANOVA). The mean values were reported to allow a comparison between the measurements of the cervical resistance at 8 mm and those of previous studies.

### Gel preparation

The gel used as placebo and the gel based on nitroprussiate were prepared at the Istituto Biochimico Italiano (Milan, Italy). Sodium nitroprussiate was supplied by Sigma (USA) and Carbopol Ultrex. 10 B.F. was supplied by Goodrich (USA). The gels at 1% and 2% by weight were prepared with the active compound. Sterility was achieved using microfilters and by operating under sterile conditions. Prefilled syringes containing nitroprussiate (0.5 g) or placebo (0.5 ml) were packed singly in plastic packages and kept in the dark at 4°C. Gels were used within a 5-month period. Gel titration carried out 6 months after preparation revealed a persistence of the NO retention activity of 95% min.

### Results

The force necessary to achieve the dilatation of the cervix from 3 to 10 mm was measured. In the subjects treated with placebo, the amount of force required increased as a function of the increase in cervix dilatation. Compared to the women treated with the placebo, those treated with the two doses of nitroprussiate gel showed significantly lower values, almost at any diameter tested. Furthermore, no difference was detected between the subjects treated with the two doses of nitroprussiate or between the two groups that received the placebo.

The resistance of the cervix at 8 mm was lower in the women that received 5 mg (24.2 ± 17, median = 24.5) or 10 mg (22.1 ± 7.6, median = 20.5) nitroprussiate gel in respect of the women that received the placebo (60.6 ± 32.6, median = 61 and 51.6 ± 28.9, median = 50). On the whole, in a sample of 36 subjects, the significant difference between intracervical nitroprussiate and placebo was 30%. Furthermore, the cumulative median force necessary to dilate the cervix to 8 mm using 5 mg to 10 mg sodium nitroprussiate (24.5 and 20.5 newtons, respectively) was much less than that detected in further trials carried out using 40 mg and 80 mg isosorbide mononitrate (40 and 36 newtons, respectively).

Table 1 reports blood pressure (BP) variations detected after treatment.

**Table 1 -**

| **BP variations (mean ± SD) after placebo or nitroprussiate application to the cervix** | | | | | | |
|---|---|---|---|---|---|---|
| | Baseline | 60 min | 120 min | 180 min | 240 min | 660 min |
| **Systolic pressure** | | | | | | |
| Placebo | 108.8 ± 7.4 | 107.2 ± 7.1 | 107.2 ± 7.5 | - | 108.3 ± 5.6 | 108.3 ± 5.6 |
| Nitropruss. 5 mg | 110.5 ± 18.5 | 103.1 ± 15.1 | 104.3 ± 17.8 | - | 107.5 ± 8.4 | 109.1 ± 8.4 |
| Placebo | 115.0 ± 9.0 | 116.1 ± 6.9 | 113.3 ± 5.6 | 113.8 ± 6.0 | - | - |
| Nitropruss. 10 mg | 110.6 ± 9.0 | 105.6 ± 9.4 | 105.0 ± 7.0 | 106.8 ± 7.5 | - | - |

| **Diastolic pressure** | | | | | | |
|---|---|---|---|---|---|---|
| Placebo | 66.1 ± 8.2 | 68.8 ± 6.9 | 67.2 ± 4.4 | - | 69.4 ± 8.8 | 70.5 ± 7.6 |
| Nitropruss. 5 mg | 69.1 ± 10.1 | 68.1 ± 12.8 | 63.7 ± 10.2 | - | 68.7 = 8.7 | 71.8 ± 7.5 |
| Placebo | 73.3 ± 7.9 | 71.6 ± 6.6 | 70.5 ± 7.6 | 73.3 ± 5.5 | - | - |
| Nitropruss. 10 mg | 63.7 ± 5.1 | 58.1 ± 9.6 | 60.0 ± 8.8 | 63.7 ± 6.9 | - | - |

According to MANOVA, no significant variations were induced by the two nitroprussiate doses (5 mg: systolic blood pressure: P = 1.91, degrees of freedom (df) 28/4; diastolic blood pressure: P = 1.48, df 28/4; 10 mg: systolic blood pressure: P = 1.44, gl 21/3; diastolic blood pressure: P = 1.34, df 21/3). In the placebo groups and in the groups treated with 5 mg and 10 mg nitroprussiate, no significant variations in systolic blood pressure (P = 0.28, df 32/4; P = 1.18, df 24/3) and in diastolic blood pressure (P = 1.60, df 32/4; P = 1.19, df 24/3) were detected. Furthermore, the onset of cephalea was not observed in the treated subjects.

The above data prove that sodium nitroprussiate applied into the cervical canal significantly softens the uterine cervix. Furthermore, notwithstanding the well known vascular activity of sodium nitroprussiate, the treatment claimed herein is substantially free from side effects, such as hypotension and chephalea. Compared with other NO donors, sodium nitroprussiate is particularly efficacious, since its effect is similar to that of prostaglandin analogue.

### Example 2: Delivery induction

### A)

It was decided to induce labour in 30 pregnant women at term (from 39 to 41+3 weeks) with reduced amniotic fluid (amniotic index < 3). The 22 cases that exhibited an unprepared cervix (Bishop score <4) received 2% NOGEL (0.5 ml) by the intracervical way. Three hours later, the Bishop score was >5 (6-10) in all cases.

The patients were then stimulated with oxytocin according to standard procedures: 18 gave birth within 12 hours, 2 within 18 hours and 2 were subjected to urgent Caesarean due to foetal reasons.

### B)

Other 20 pregnant women, who exhibited characteristics analogous to those reported above, received 1 % NOGEL (1 ml) in the vagina; stimulation with oxytocin started 6 hours later. Cervical ripening (from Bishop <3 to Bishop>5) took place in 17 cases: out of them 13 gave birth within 12 hours after oxytocin stimulus and the remaining 4 within 24 hours.

### Example 3: Abortion induction in the 2nd trimester

In 21 pregnant women at 13 to 22 weeks, pregnancy was ended at request of the mothers (presence of malformations/psychic disorders: 6 cases) and due to reasons connected with the feto-placental unit (membrane rupture, endouterine death: 15 cases). All patients exhibited an unprepared, rigid and closed cervix (Bishop score <3).

According to a random scheme, 10 cases were treated only with intravenous prostaglandins (Nalador - Schering) following standard clinical procedures, and 11 cases were pretreated with intracervical NOGEL (0.5 ml) and, immediately after, were stimulated with Nalador.

In NOGEL-pretreated patients, expulsion of the contents of the uterus took place after 2 to 9 hours (average: 7.1 hours), whereas in the other patients, expulsion took place after 5 to 22 hours (average: 14.3 hours), with significantly longer times (p<0.01).

### Example 4: Revision of the uterine cavity in the 1st trimester

In 14 pregnant women that had a miscarriage (missed or incomplete) at 6 to 11 weeks, 1% NOGEL (0.5 ml) was applied by the intracervical way, prior to the instrumental revision of the uterine cavity, which was carried out two hours later under local anaesthesia, by pudenda block with carbocaine.

In all but 2 cases, no dilatation of the cervix by means of Hegar's dilators was necessary, and the uterus suction and curretage were performed directly. Controls performed in the successive 24 hours showed no anomalous bleeding from the patients' genitals.

### Example 5: Assisted procreation

Thirty patients, 29 to 44 years old, were subjected to embryos transfer, after *in vitro* fertilisation, as required for infertility. Out of them, 15 received 1% NOGEL (0.2 ml) one hour before insertion of the cervical catheter and 15 received the placebo. In NOGEL group, all inseminations did not present any difficulty, whereas in 2 cases treated with placebo, insemination had to be discontinued and, to proceed, the cervix had to be dilated mechanically.

### Example 6: Cavity examination/Endometrial curettage

Forty postmenopausal patients, 54 to 79 years old, hospitalised for menorrhagia, needed endometrial biopsy with examination of the uterine cavity.

Two hours before the operation, 20 cases were treated with 0.5 ml placebo gel and 20 cases with 2% NOGEL (0.5 ml), by the intracervical way.

Prior to the operation, the cervix resistance to the penetration of a dilator sized 6 mm--i.e. having the same diameter as the curette used for scraping out the endometrium--was tested. The mean force required in the placebo group was 45 newtons and in the NOGEL group was 13 newtons (P<0.001).

In 10 out of the 20 cases treated with NOGEL, no cervix dilatation was necessary to proceed with uterine cavity examination.

### Example 7: Insertion of intra-uterine devices (IUD)

Nine cases were characterised by difficult IUD insertion due to cervix resistance.

The first attempt of insertion failed. One to three months later, said women were treated--prior to the 2nd attempt--with 2% NOGEL (0.5 ml) by the intracervical way. One hour later, the IUD was successfully inserted in all cases.

### Example 8: Hysteroscopy

Studies were conducted on 14 patients suffering from endometrial polyps and/or submucous myomas. Visualisation of the uterine cavity by a hysteroscope was required. Three hours before the examination, seven cases were treated with 1% NOGEL (1 ml) by the vaginal way. Untreated women had to be subjected to mechanical dilatation of the cervix (Hegar's dilator No. 6); instead, in the women treated with NOGEL, the hysteroscope was inserted without any difficulty and the examination was brought to a successful conclusion without cervix dilatation.

Examples 2 to 8 show that the treatment with nitroprussiate may be used successfully in a large number of obstetric and gynaecological operations, which require an increased uterus distensibility, softness and penetrability.

## Claims

1. Use of sodium nitroprussiate in the preparation of a drug useful in obstetric and gynaecological treatment.

2. The use as claimed in claim 1, wherein said drug is useful in procedures requiring an increased distensibility, softness and penetrability of the uterine cervix.

3. The use as claimed in claim 2, wherein said procedures include artificial insemination, natural or induced delivery, insertion of uterine devices, performance of gynaecological treatment or tests, such as for example hysteroscopy, revision of the uterine cavity, suction of the uterus, the endometrial curettage and abortion induction.

4. The use as claimed in any of claims 1 to 3, wherein said drug is suitable for intracervical or vaginal administration.

5. The use as claimed in claim 4, wherein said drug is in the form of ointment, gel, lavage, tablet, suppository, medicated aid.

6. The use as claimed in any of claims 1 to 5, wherein nitroprussiate is administered in an amount of 1 mg to 10 mg.

7. Pharmaceutical composition suitable for intracervical or vaginal administration, including sodium nitroprussiate combined with suitable excipients and diluents for pharmaceutical use.

8. The composition as claimed in claim 7 in the form of ointment, gel, lavage, tablet, suppository, medicated aid.

9. The composition as claimed in any of claims 7 to 8 containing sodium nitroprussiate in a concentration comprised between 0.5% and 3% by weight.
